# EUROPÄISCHE PATENTANMELDUNG

(11) **EP 2 623 134 A1**
(43) Veröffentlichungstag der Anmeldung: **07.08.2013**
(21) Anmeldenummer: 12000658.0
(22) Anmeldetag: 01.02.2012
(51) Int. Cl.: A61L 27/26, A61L 27/56, A61L 27/58

(54) **Implantat-matrix aus einem Polymergemisch**

(71) Anmelder: PHRONTIER S.A.R.L., 76490 Caudebec-en-Caux (FR)
(72) Erfinder: Görne, Martin, DE-22337 Hamburg (DE)
(74) Vertreter: Diehl & Partner GbR

(57) **Zusammenfassung**

Eine Implantat-Matrix besteht überwiegend aus einem Gemisch von unterschiedlich schnell abbaubaren Polymeren, wobei sich nominelle Resorptionszeiten von zwei der Gemischkomponenten, die jeweils mindestens 10 % des Gemisches ausmachen, um einen Faktor von mindestens 5 unterscheiden. Die Implantat-Matrix wird aus einem Gemisch der mindestens zwei unterschiedlich schnell abbaubaren Polymere hergestellt, wobei Partikel der beiden Polymere mit Partikeln eines wasserlöslichen Feststoffs und einem Lösungsmittel für eines der Polymere gemischt und nach Abdampfen des Lösungsmittels gewünschtenfalls kompaktiert werden, und der Feststoff dann durch Wässern entfernt wird.

## Beschreibung

Die vorliegende Anmeldung bezieht sich auf poröse Matrices für chirurgische Zwecke.

Zellimplantate auf der Basis poröser Matrices aus bioverträglichen Polymeren sind aus WO 2004/108810 Al bekannt. Bei solchen Matrices sind die Poren vernetzt und dienen als Templat für die Ansiedlung von Zellen *in vivo* (z. B. therapeutisch) oder *in vitro* (z. B. diagnostisch). Bei Transplantationen kann eine solche bioresorbierbare Matrix zur temporären Lokalisation des Transplantats und als Platzhalter für sich nach und nach bildendes Gewebe dienen.

Die bekannten Template sind bei einigen Anwendungen noch nicht voll befriedigend, insbesondere hinsichtlich der klinischen Ergebnisse.

Die Erfindung setzt sich daher zum Ziel, eine Verbesserung der klinischen Performance der Template zu erreichen.

Dazu schlägt die Erfindung ein poröses Templat aus einem Gemisch von unterschiedlich schnell abbaubaren Polymeren vor, wobei sich nominelle Resorptionszeiten von zwei der Gemischkomponenten, die jeweils mindestens 10% des Gemisches ausmachen, um einen Faktor von mindestens 5 unterscheiden. Ohne Beschränkung auf die vermutete Wirkungsweise nehmen die Erfinder an, dass das schneller resorbiert werdende Polymer nach und nach Platz schafft für die sich bildenden Gefäße, während der Zusammenhalt der Gesamtstruktur durch das langsamer resorbiert werdende Polymer gewährleistet ist, ohne dass einzelne Strukturelemente als Fremdkörper wirken. Zudem verändert der fortschreitende Abbau des schneller resorbiert werdenden Polymers das physiologische Milieu in für den therapeutischen Erfolg günstiger Weise.

Unter einem weiteren Aspekt schlägt die Erfindung Verfahren zur Herstellung von porösen bioresorbierbaren Matrices vor, wobei ein Gemisch aus mindestens zwei unterschiedlich schnell resorbierbaren Polymeren und einem wasserlöslichen Porenbildner und einem Lösungsmittel für eines der Polymere hergestellt, gefolgt von Abdampfen des Lösungsmittels und Wässern zur Porenbildung. In Varianten wird das Gemisch nach dem Abdampfen des Lösungsmittels kompaktiert. Beide Verfahren führen zu hochporösen Polymermatrixscheiben, deren klinische Performance hervorragend ist. Die Abbauzeiten der Polymere unterscheiden sich um einen Faktor 5 oder mehr.

Weitere Merkmale der Erfindung ergeben sich aus der nachfolgenden Beschreibung von Ausführungsbeispielen in Verbindung mit den Ansprüchen sowie den Figuren. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein.

In einer Hauptanwendung werden Matrices zur Defektdeckung bereitgestellt, beispielsweise einer Hernien-Dehiszenz. Es ist vorgesehen, dass ein erster Teil der verwendeten Polymermischung schneller abgebaut wird und ein anderer Teil der Polymermischung langsamer erodiert (Verhältnis der Abbauzeiten mindestens 5) und den strukturellen Zusammenhalt längere Zeit, z. B. 2,5-3 Jahre (oder mindestens 2 und/oder weniger als 5 Jahre) gewährleistet. Durch die allmähliche Auflösung des schneller abbaubaren Teils der Matrix binnen 3-4 Monaten, oder mindestens 2 und/oder weniger als 7 Monaten wird das physiologische Milieu in einer Weise beeinflusst, die dem therapeutischen Erfolg dienlich ist. Solche Polymere sind zweckmäßig auf Basis von α-Hydroxycarbonsäuren wie Milchsäure und/oder Glykolsäure, z. B. PLA oder PLGA. Die Hersteller solcher zum Einsatz im menschlichen Körper zugelassener Polymere geben die hier relevanten nominellen Abbauzeiten an. Die hier verwendeten Polymere sind z. B. von der Fa. Evonik erhältlich und tragen die Bezeichnungen L210s, L210, L09s, L207s, L206s (langsamer abbaubare PLGA-Polymere) bzw. RG502, RG502H, RG505 (schneller abbaubare PLGA-Polymere).

In der Hauptvariante werden die erfindungsgemäßen Matrices mechanisch hinreichend stabil hergestellt, dass sie z. B. den Belastungen durch chirurgische Nähvorgänge standhalten. An ihrer Peripherie können die Matrices dadurch mit Körpergewebe verbunden werden. Ihre Porosität stellt sicher, dass die Matrices mit Bindegewebszellen infiltriert werden.

Gemäß einer Ausführungsform weist die Matrix eine porenarme oder -frei Seite auf, die für die eigentliche Abdeckung sorgt, und eine porenreiche, die für die Infiltration günstig ist. Im Körper kann dabei die glattere porenarme Seite dem Körperinneren zugewandt angeordnet werden, um bei Ausübung von Druck durch die Körperorgane auf die Defektstelle keine Angriffsstelle zu bieten.

In einer Variante wird die Matrix vorab beispielsweise mit Hepatozyten und/oder mit Langerhans'schen Inselzellen infiltriert. Solche biochemischen Funktionszellen heften sich an die Innenwände der Poren der schaumartigen Matrix an (Anheftungsraten über 80 % oder, geeignet beschichtet, über 95 %) und können mit der Matrix in mesotheliale Taschen transplantiert werden, idealerweise des Zellspenders selbst. Dabei wird ausgenutzt, dass in diesem Fall keine Abstoßungsreaktion erfolgt, sondern nur ein vergleichsweise milder, für den therapeutischen Prozess günstiger Fremdkörper-Reiz ausgeübt wird. Binnen weniger Wochen wird die Matrix vaskularisiert und sind die implantierten Zellen nicht mehr nur auf diffusive Versorgung angewiesen. Die Matrices werden so angeordnet, dass die porenarme (oder -freie) Seite innen und die porenreiche Seite außen liegt, um die Verlustrate durch Abwanderung der Zellen niedrig zu halten.

Zunächst wird in einer Ausführungsform eine Lösung eines der verwendeten Polymere in für medizinische Zwecke zugelassenem Chloroform in eine Form gegossen und das Lösungsmittel bei 45°-65° abgedampft. Daraufhin wird eine Polymer-Mischung definierter Partikelgrößen-Verteilung mit einem Kochsalz-Granulat ebenfalls definierter Partikelgrößen-Verteilung gemischt, mit einer Lösung eines der Polymere in Chloroform vermengt und dann auf die bereits hergestellte Polymerschicht gegeben. Aus diesem Vorformling dampft das Lösungsmittel bei leicht erhöhter Temperatur (45-65°C) ab; sodann kann er gewünschtenfalls durch Beaufschlagung mit Druck kompaktiert werden. Anschließend wird der Kompaktkörper gewässert, um durch Entfernen des Salzes die gewünschte Porosität bereitzustellen. Dabei bleibt eine initial hergestellte Polymerschicht porenfrei. Je nach Einsatzzweck kann die Dicke der porenarmen Schicht durch Menge und Konzentration der anfänglichen Lösung eingestellt werden. Beispielsweise erhält man eine stabile Membran, wenn bei einer Konzentration der Lösung von z. B. 4 % in Chloroform (langsam abbaubares Polymer) die Füllhöhe etwa 5-50 mm, typisch 20-25 mm beträgt. Das Abdampfen des Chloroforms dauert dann etwa 1,5 h und resultiert in einer Schichtdicke von ca. 0,5-2 mm. Im anderen Fall geht man bei gleicher Polymer-Konzentration von einer Füllhöhe von nur 0,1-0,5 mm aus, wodurch das Abdampfen des Chloroforms schneller beendet ist (ca. 20-30 min), und die entstehende Membran eine Dicke von nur ca. 10-20 µm hat.

Die Kochsalzpartikel der porenbildenden Mischung sind etwas gröber (Median bei 400-420 µm) als die Polymerpartikel (Median des langsamer abbaubaren Polymers zwischen 210 µm und 230 µm, der des schneller abbaubaren Polymers zwischen 150 µm und 170 µm). Dabei sind die Verteilungsbreiten (5 %/95 %) ähnlich, nämlich etwa ± 85-95 µm für Salz bzw. Polymer insgesamt. Die Verteilungsform kann bi- oder trimodal sein. Die Zusammensetzung der Schichtmischung ist zu etwa 96 % Salz, 1-1,5 % festes Polymer und weitere ca. 3-5 % gelöstes Polymer, wobei die Volumenanteile von Feststoffen und Flüssigkeit etwa gleich sind. Insgesamt beträgt der Anteil des schnell abbaubaren Polymers lediglich etwa 5-20% des Polymers. Die Gesamtdicke der porenbildenden Schicht ist 4-5 mm. In der Variante einer fragileren Initialschicht kann das Salz etwas feiner gewählt werden (Median ca. 350-370 µm). In diesem Fall ist die Gesamtdicke der porenbildenden Schicht 5-6 mm. Das Wässern dauert ca. 24 h und wird gefolgt von einer Trocknung bei 45-50°C.

Bei einer Verwendung außerhalb des Körpers kann eine Matrix gemäß der obigen Beschreibung zur Fixierung von Zellen dienen, die in einem Bioreaktor Agenzien ausgesetzt werden. Beispielsweise können definierte Zelltypen auf diese Weise daraufhin untersucht werden, ob sie auf in Frage kommende Medikamente ansprechen oder nicht, und kann die Therapie in Abhängigkeit von derart erhaltenen Untersuchungsergebnissen geplant werden. Ebenso kann die Medikamentenentwicklung vereinfacht werden, weil Toxizität frühzeitig erkannt wird.

Der Fachmann wird erkennen, dass im Rahmen des Schutzumfangs der beigefügten Ansprüche Abwandlungen der oben beschriebenen Beispiele möglich sind.

## Patentansprüche

1. Implantat-Matrix, die überwiegend aus einem Gemisch von unterschiedlich schnell abbaubaren Polymeren besteht, wobei sich nominelle Resorptionszeiten von zwei der Gemischkomponenten, die jeweils mindestens 10% des Gemisches ausmachen, um einen Faktor von mindestens 5 unterscheiden.

2. Implantat-Matrix nach Anspruch 1, wobei die Gemischkomponenten Poly(α-hydroxy)carbonsäuren wie PLA und PLGA sind.

3. Implantat-Matrix nach Anspruch 1 oder 2, wobei die Matrix eine Porosität von mindestens 80 % aufweist.

4. Implantat-Matrix nach einem der vorstehenden Ansprüche, wobei eine nominelle Resorptionszeit des schneller abbaubaren Polymers weniger als 4 Monate beträgt.

5. Implantat-Matrix nach einem der vorstehenden Ansprüche, wobei eine nominelle Resorptionszeit des langsamer abbaubaren Polymers mehr als 20 Monate beträgt.

6. Verwendung der Matrix nach einem der vorstehenden Ansprüche zum Besiedeln mit vitalen Zellen, und zum Aussetzen der Zellen einem vorbestimmten Test-Agens.

7. Verfahren zur Herstellung einer Implantat-Matrix aus einem Gemisch von mindestens zwei unterschiedlich abbaubaren Polymeren, wobei Partikel der beiden Polymere mit Partikeln eines wasserlöslichen Feststoffs und einer Lösung wenigstens eines der Polymere in einem mit Wasser nicht mischbaren Lösungsmittel gemischt werden, und der Feststoff dann durch Wässern entfernt wird, wobei sich nominelle Resorptionszeiten der Gemischkomponenten, die jeweils mindestens 10 % des Gemisches ausmachen, um einen Faktor von mindestens 5 unterscheiden.

8. Verfahren nach Anspruch 7, umfassend Beaufschlagen mit Druck zum Kompaktieren des Gemischs nach dem Abdampfen des Lösungsmittels und vor dem Wässern.

9. Verfahren nach Anspruch 7 oder 8, wobei als Lösungsmittel Chloroform verwendet wird.

10. Verfahren nach einem der Ansprüche 7 bis 9, ferner umfassen Besiedeln der Matrix mit vitalen Zellen außerhalb des Körpers, und Aussetzen der Zellen einem vorbestimmten Test-Agens.
